(19) Europäisches Patentamt
European Patent Office
Office européen des brevets

(11) **EP 3 427 797 A1**

(12) **EUROPEAN PATENT APPLICATION**

(43) Date of publication:
**16.01.2019 Bulletin 2019/03**

(51) Int Cl.:
*A61P 35/00* (2006.01)   *A61K 31/5377* (2006.01)

(21) Application number: **18187344.9**

(22) Date of filing: **10.10.2008**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **12.10.2007   EP 07118421**

(62) Document number(s) of the earlier application(s) in accordance with Art. 76 EPC:
**08836801.4 / 2 209 529**

(71) Applicant: **Vernalis (R&D) Limited
Winnersh
Wokingham
Berkshire RG41 5RD (GB)**

(72) Inventors:
• **CHENE, Patrick
Wokingham, Berkshire RG41 5RD (GB)**

• **GARCIA-ECHEVERRIA, Carlos
Wokingham, Berkshire RG41 5RD (GB)**
• **JENSEN, Michael Rugaard
Wokingham, Berkshire RG41 5RD (GB)**
• **QUADT, Cornelia
Wokingham, Berkshire RG41 5RD (GB)**
• **RADIMERSKI, Thomas
Wokingham, Berkshire RG41 5RD (GB)**
• **SCHOEPFER, Joseph
Wokingham, Berkshire RG41 5RD (GB)**

(74) Representative: **Gill Jennings & Every LLP
The Broadgate Tower
20 Primrose Street
London EC2A 2ES (GB)**

Remarks:
This application was filed on 03-08-2018 as a divisional application to the application mentioned under INID code 62.

(54) **ISOXAZOLE COMPOUND FOR THE TREATMENT OF LUNG CANCER**

(57)   The use of 5-(2,4-Dihydroxy-5-isopropyl-phenyl)-4-(4-morpholin-4-ylmethyl-phenyl)isoxazole-3-carboxylic acid ethylamide or a pharmaceutically acceptable salt, hydrate or solvate thereof, for the manufacture of a pharmaceutical composition for the treatment of cancer of the lung.

EP 3 427 797 A1

**Description**

The invention relates to the use of 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate for the manufacture of pharmaceutical compositions for use in the treatment of cancer, e.g. solid tumors, e.g. sarcomas, e.g. carcinomas of the bladder, the colon, the liver, the lung, e.g. pleural mesothelioma, e.g. non small cell, e.g. small cell, the breast, the vagina, the ovaries, the pancreas, the kidney, the stomach, gastrointestinal tract, e.g. gastrointestinal stromal tumor, e.g. the small intestine, e.g. the esophagus, e.g. the bile duct, the prostate, the head and neck, the peritoneal cavity, the thyroid, the bone, the brain, the central nervous system e.g. glioblastoma, e.g. neuroblastoma, and/or melanoma and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or for use in treatment of myelodysplastic syndrome, systemic mastocytosis, von Hippel-Lindau syndrome, multicentric Castleman disease and/or psioriasis, to the use of 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate in the treatment of cancer, e.g. solid tumors, e.g. sarcomas, e.g. carcinomas of the bladder, the colon, the liver, the lung, e.g. pleural mesothelioma, e.g. non small cell, e.g. small cell, the breast, the vagina, the ovaries, the pancreas, the kidney, the stomach, gastrointestinal tract, e.g. gastrointestinal stromal tumor, e.g. the small intestine, e.g. the esophagus, e.g. the bile duct, the prostate, the head and neck, the peritoneal cavity, the thyroid, the bone, the brain, the central nervous system e.g. glioblastoma, e.g. neuroblastoma, and/or melanoma and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or in the treatment of myelodysplastic syndrome, systemic mastocytosis, von Hippel-Lindau syndrome, multicentric Castleman disease and/or psioriasis, and to a method of treating warm-blooded animals including humans suffering from cancer, e.g. solid tumors, e.g. sarcomas, e.g. carcinomas of the bladder, the colon, the liver, the lung, e.g. pleural mesothelioma, e.g. non small cell, e.g. small cell, the breast, the vagina, the ovaries, the pancreas, the kidney, the stomach, gastrointestinal tract, e.g. gastrointestinal stromal tumor, e.g. the small intestine, e.g. the esophagus, e.g. the bile duct, the prostate, the head and neck, the peritoneal cavity, the thyroid, the bone, the brain, the central nervous system e.g. glioblastoma, e.g. neuroblastoma, and/or melanoma and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or myelodysplastic syndrome, systemic mastocytosis, von Hippel-Lindau syndrome, multicentric Castleman disease and/or psioriasis by administering to said animal in need of such treatment an effective dose of 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate.

Management of cancer, e.g. solid tumors, e.g. sarcomas, e.g. carcinomas of the bladder, the colon, the liver, the lung, e.g. pleural mesothelioma, e.g. non small cell, e.g. small cell, the breast, the vagina, the ovaries, the pancreas, the kidney, the stomach, gastrointestinal tract, e.g. gastrointestinal stromal tumor, e.g. the small intestine, e.g. the esophagus, e.g. the bile duct, the prostate, the head and neck, the peritoneal cavity, the thyroid, the bone, the brain, the central nervous system e.g. glioblastoma, e.g. neuroblastoma, and/or melanoma and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or myelodysplastic syndrome, systemic mastocytosis, von Hippel-Lindau syndrome, multicentric Castleman disease and/or psioriasis is a major problem.

Heat shock protein 90 (Hsp90) is recognized as a new anti-cancer target. Hsp90 is a ubiquitous, highly abundant (1-2% of the total cellular protein), essential protein which functions as a molecular chaperone to ensure the conformational stability, shape and function of client proteins. Inhibition of its intrinsic ATPase activity of Hsp90 disrupts the Hsp90-client protein interaction resulting in their degradation via the ubiquitin proteasome pathway. A subset of Hsp90 client proteins, such as Raf, AKT, CDK4 and the EGFR family including ErbB2 are oncogenic signaling molecules critically involved in cell growth, differentiation and apoptosis, processes which are fundamentally important in cancer cells. The simultaneous degradation of multiple oncoproteins is believed to produce the anti-tumor effects observed with Hsp90 inhibitors.

The Hsp90 family of chaperones is comprised of four members: Hsp90$\alpha$ and Hsp90$\beta$ both located in the cytosol, GRP94 in the endoplasmic reticulum, and TRAP1 in the mitochondria (Csermely et al., 1998). Hsp90 is the most abundant cellular chaperone, constituting about 1% - 2% of total protein (Jakob and Buchner, 1994). Among the stress proteins, Hsp90 is unique because it is not required for the biogenesis of most polypeptides (Nathan et al., 1997). Its cellular targets, also called client proteins, are conformationally labile signal transducers that play a critical role in growth control, cell survival and tissue development (Pratt and Toft, 2003).

Hsp90 chaperones, which possess a conserved ATP-binding site at their N-terminal domain (Chene, 2002) belong to a small ATPase sub-family known as the DNA Gyrase, Hsp90, Histidine Kinase and MutL (GHKL) sub-family (Dutta and Inouye, 2000). The chaperoning (folding) activity of Hsp90 depends on its ATPase activity which is weak for the isolated enzyme. However, it has been shown that the ATPase activity of Hsp90 is enhanced upon its association with proteins known as co-chaperones (Kamal et al., 2003). Therefore, *in vivo*, Hsp90 proteins work as subunits of large, dynamic protein complexes. Hsp90 is essential for eukaryotic cell survival and is overexpressed in many tumors.

5-(2,4-Dihydroxy-5-isopropyl-phenyl)-4-(4-morpholin-4-ylmethyl-phenyl)-isoxazole-3-carboxylic acid ethylamide is an Hsp90 inhibitor, its synthesis is described for instance in WO 2004/072051, example 78, included herein by reference.

Surprisingly it has now been found that 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate is useful in the treatment of cancer, e.g. solid tumors, e.g. sarcomas, e.g. carcinomas of the bladder, the colon, the liver, the lung, e.g. pleural mesothelioma, e.g. non small cell, e.g. small cell, the breast, the vagina, the ovaries, the pancreas, the kidney, the stomach, gastrointestinal tract, e.g. gastrointestinal stromal tumor, e.g. the small intestine, e.g. the esophagus, e.g. the bile duct, the prostate, the head and neck, the peritoneal cavity, the thyroid, the bone, the brain, the central nervous system e.g. glioblastoma, e.g. neuroblastoma, and/or melanoma and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or in treating of myelodysplastic syndrome, systemic mastocytosis, von Hippel-Lindau syndrome, multicentric Castleman disease and/or psioriasis.

Accordingly the present invention provides the use of 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate for the manufacture of pharmaceutical compositions for use in the treatment of cancer, e.g. solid tumors, e.g. sarcomas, e.g. carcinomas of the bladder, the colon, the liver, the lung, e.g. pleural mesothelioma, e.g. non small cell, e.g. small cell, the breast, the vagina, the ovaries, the pancreas, the kidney, the stomach, gastrointestinal tract, e.g. gastrointestinal stromal tumor, e.g. the small intestine, e.g. the esophagus, e.g. the bile duct, the prostate, the head and neck, the peritoneal cavity, the thyroid, the bone, the brain, the central nervous system e.g. glioblastoma, e.g. neuroblastoma, and/or melanoma and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or for use in treatment of myelodysplastic syndrome, systemic mastocytosis, von Hippel-Lindau syndrome, multicentric Castleman disease and/or psioriasis.

In another aspect the present invention provides the use of 5-(2,4-Dihydroxy-5-isopropylphenyl)-4-(4-morpholin-4-ylmethyl-phenyl)-isoxazole-3-carboxylic acid ethylamide or a tautomer thereof or a pharmaceutically acceptable salt or a hydrate or a solvate in the treatment of cancer, e.g. solid tumors, e.g. sarcomas, e.g. carcinomas of the bladder, the colon, the liver, the lung, e.g. pleural mesothelioma, e.g. non small cell, e.g. small cell, the breast, the vagina, the ovaries, the pancreas, the kidney, the stomach, gastrointestinal tract, e.g. gastrointestinal stromal tumor, e.g. the small intestine, e.g. the esophagus, e.g. the bile duct, the prostate, the head and neck, the peritoneal cavity, the thyroid, the bone, the brain, the central nervous system e.g. glioblastoma, e.g. neuroblastoma, and/or melanoma and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or in the treatment of myelodysplastic syndrome, systemic mastocytosis, von Hippel-Lindau syndrome, multicentric Castleman disease and/or psioriasis.

In a further aspect the present invention provides 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate for use in treating cancer, e.g. solid tumors, e.g. sarcomas, e.g. carcinomas of the bladder, the colon, the liver, the lung, e.g. pleural mesothelioma, e.g. non small cell, e.g. small cell, the breast, the vagina, the ovaries, the pancreas, the kidney, the stomach, gastrointestinal tract, e.g. gastrointestinal stromal tumor, e.g. the small intestine, e.g. the esophagus, e.g. the bile duct, the prostate, the head and neck, the peritoneal cavity, the thyroid, the bone, the brain, the central nervous system e.g. glioblastoma, e.g. neuroblastoma, and/or melanoma and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or for use in treating myelodysplastic syndrome, systemic mastocytosis, von Hippel-Lindau syndrome, multicentric Castleman disease and/or psioriasis.

In a further aspect the present invention provides a method of treating humans suffering from cancer, e.g. solid tumors, e.g. sarcomas, e.g. carcinomas of the bladder, the colon, the liver, the lung, e.g. pleural mesothelioma, e.g. non small cell, e.g. small cell, the breast, the vagina, the ovaries, the pancreas, the kidney, the stomach, gastrointestinal tract, e.g. gastrointestinal stromal tumor, e.g. the small intestine, e.g. the esophagus, e.g. the bile duct, the prostate, the head and neck, the peritoneal cavity, the thyroid, the bone, the brain, the central nervous system e.g. glioblastoma, e.g. neuroblastoma, and/or melanoma and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g.

multiple myeloma e.g. lymphomas, and/or myelodysplastic syndrome, systemic mastocytosis, von Hippel-Lindau syndrome, multicentric Castleman disease and/or psioriasis which comprises administering to said human in need of such treatment a dose of 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate effective against cancer, e.g. solid tumors, e.g. sarcomas, e.g. carcinomas of the bladder, the colon, the liver, the lung, e.g. pleural mesothelioma, e.g. non small cell, e.g. small cell, the breast, the vagina, the ovaries, the pancreas, the kidney, the stomach, gastrointestinal tract, e.g. gastrointestinal stromal tumor, e.g. the small intestine, e.g. the esophagus, e.g. the bile duct, the prostate, the head and neck, the peritoneal cavity, the thyroid, the bone, the brain, the central nervous system e.g. glioblastoma, e.g. neuroblastoma, and/or melanoma and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or myelodysplastic syndrome, systemic mastocytosis, von Hippel-Lindau syndrome, multicentric Castleman disease and/or psioriasis.

In a further aspect the present invention provides a pharmaceutical preparation for the treatment of cancer, e.g. solid tumors, e.g. sarcomas, e.g. carcinomas of the bladder, the colon, the liver, the lung, e.g. pleural mesothelioma, e.g. non small cell, e.g. small cell, the breast, the vagina, the ovaries, the pancreas, the kidney, the stomach, gastrointestinal tract, e.g. gastrointestinal stromal tumor, e.g. the small intestine, e.g. the esophagus, e.g. the bile duct, the prostate, the head and neck, the peritoneal cavity, the thyroid, the bone, the brain, the central nervous system e.g. glioblastoma, e.g. neuroblastoma, and/or melanoma and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or myelodysplastic syndrome, systemic mastocytosis, von Hippel-Lindau syndrome, multicentric Castleman disease and/or psioriasis comprising 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate and at least one pharmaceutically acceptable carrier.

Depending on species, age, individual condition, mode of administration, and the clinical picture in question, effective doses for example weekly doses of about 2 to 300 mg, preferably 50 to 160 mg of 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate are administered to a human.

The present invention further provides a method for administering to a human having cancer, e.g. solid tumors, e.g. sarcomas, e.g. carcinomas of the bladder, the colon, the liver, the lung, e.g. pleural mesothelioma, e.g. non small cell, e.g. small cell, the breast, the vagina, the ovaries, the pancreas, the kidney, the stomach, gastrointestinal tract, e.g. gastrointestinal stromal tumor, e.g. the small intestine, e.g. the esophagus, e.g. the bile duct, the prostate, the head and neck, the peritoneal cavity, the thyroid, the bone, the brain, the central nervous system e.g. glioblastoma, e.g. neuroblastoma, and/or melanoma and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or myelodysplastic syndrome, systemic mastocytosis, von Hippel-Lindau syndrome, multicentric Castleman disease and/or psioriasis 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate, which comprises administering a pharmaceutically effective amount of 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate to a human subject about once weekly or more frequently.

Numbered Embodiments

1. The use of 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate for the manufacture of pharmaceutical compositions for use in the treatment of cancer of the bladder, the colon, the liver, the lung, the breast, the vagina, the ovaries, the pancreas, the kidney, the stomach, the gastrointestinal tract, the prostate, the head and neck, the peritoneal cavity, the thyroid, the bone, the brain, the central nervous system and/or the blood and/or myelodysplastic syndrome, systemic mastocytosis, von Hippel-Lindau syndrome, multicentric Castleman disease and/or psioriasis.

2. The use of 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate in the treatment of cancer of the bladder, the colon, the liver, the lung, the breast, the vagina, the ovaries, the pancreas, the kidney, the stomach, the gastrointestinal tract, the prostate, the head and neck, the peritoneal cavity, the thyroid, the bone, the brain, the central nervous system and/or the blood and/or myelodysplastic syndrome, systemic mastocytosis, von Hippel-Lindau syndrome, multicentric Castleman disease and/or psioriasis.

3. A method of treating humans suffering from cancer of the bladder, the colon, the liver, the lung, the breast, the ovaries, the pancreas, the kidney, the stomach, the gastrointestinal tract, the prostate, the head and neck, the brain, and/or the blood which comprises administering to said human in need of such treatment a dose of 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate, effective against cancer of the bladder, the colon, the liver, the lung, the breast, the vagina, the ovaries, the pancreas, the kidney, the stomach, the gastrointestinal tract, the prostate, the head and neck, the peritoneal cavity, the thyroid, the bone, the brain, the central nervous system and/or the blood and/or myelodysplastic syndrome, systemic mastocytosis, von Hippel-Lindau syndrome, multicentric Castleman disease and/or psioriasis.

4. A pharmaceutical preparation for the treatment of cancer of the bladder, the colon, the liver, the lung,the breast, the vagina, the ovaries, the pancreas, the kidney, the stomach, the gastrointestinal tract, the prostate, the head and neck, the peritoneal cavity, the thyroid, the bone, the brain, the central nervous system and/or the blood and/or myelodysplastic syndrome, systemic mastocytosis, von Hippel-Lindau syndrome, multicentric Castleman disease and/or psioriasis comprising 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate and at least one pharmaceutically acceptable carrier.

5. Use or method according to any one of numbered embodiments 1 to 3, wherein a weekly dose of 2 to 300 mg of 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate is administered to a human.

6. A method for administering to a human subject having cancer of the bladder, the colon, the liver, the lung,the breast, the vagina, the ovaries, the pancreas, the kidney, the stomach, the gastrointestinal tract, the prostate, the head and neck, the peritoneal cavity, the thyroid, the bone, the brain, the central nervous system and/or the blood and/or myelodysplastic syndrome, systemic mastocytosis, von Hippel-Lindau syndrome, multicentric Castleman disease and/or psioriasis 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate, which comprises administering a pharmaceutically effective amount of 5-(2,4-Dihydroxy-5-isopropylphenyl)-4-(4-morpholin-4-ylmethyl-phenyl)-isoxazole-3-carboxylic acid ethylamide or a tautomer thereof or a pharmaceutically acceptable salt or a hydrate or a solvate to the human subject once weekly or more frequently.

7. 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate in use for treating cancer of the bladder, the colon, the liver, the lung, the breast, the vagina, the ovaries, the pancreas, the kidney, the stomach, the gastrointestinal tract, the prostate, the head and neck, the peritoneal cavity, the thyroid, the bone, the brain, the central nervous system and/or the blood and/or myelodysplastic syndrome, systemic mastocytosis, von Hippel-Lindau syndrome, multicentric Castleman disease and/or psioriasis.

Following is a description by way of example only.

**Example 1: *In vitro* effects of 5-(2,4-Dihydroxy-5-isopropyl-phenyl)-4-(4-morpholin-4-ylmethyl-phenyl)-isoxazole-3-carboxylic acid ethylamide (AUY922) on a panel of tumor derived cell lines.**

Thirty-seven cancer derived cell lines are used (BT474, MDA-MB-361, MDA-MB-453, SKBr3, T47D, MCF7, MDA-MB-231, MDA-MB-468, SK-MEL-5, A375, MALME-3M, SK-MEL-28, WM266.4, RPMI8226, U266, BE, Colo205, HCT116, HT29, MAWI, RKO, U87MG, HN5, RPMI-8226, A549, MV522, NCI-H1299, NCI-H460, 41M, A2780, CH1, NCI-N87, SKOV3, PC3, MO7e, GIST882 and Baf3) to test the effect of 5-(2,4-Dihydroxy-5-isopropyl-phenyl)-4-(4-morpholin-4-ylmethyl-phenyl)-isoxazole-3-carboxylic acid ethylamide. Cell lines are commercially available from American Type Culture Collection (ATCC). These cell lines cover the following 12 cancer or tumor types: breast, melanoma, multiple myeloma (MM), colon, glioblastoma, head & neck, leukemia, lung, ovarian, prostate, stomach and gastrointestinal stromal tumour (GIST). After division and medium change, cells from stock culture are seeded on cell plates and cultured for about 18 hours to allow cell growth and attachment before starting the assay. On the first day of the assay, 5-(2,4-Dihydroxy-5-isopropyl-phenyl)-4-(4-morpholin-4-ylmethyl-phenyl)-isoxazole-3-carboxylic acid ethylamide is added to the medium at various concentrations up to 10 $\mu$. Cells are cultured up to 72 or 96 hours and cell proliferation is determined using commercially available cell proliferation kits.

Table 1 shows the concentrations (nM) of 5-(2,4-Dihydroxy-5-isopropyl-phenyl)-4-(4-morpholin-4-ylmethyl-phenyl)-isoxazole-3-carboxylic acid ethylamide which inhibit cell proliferation by 50% ($IC_{50}$). The cells were continually exposed to 5-(2,4-Dihydroxy-5-isopropyl-phenyl)-4-(4-morpholin-4-ylmethyl-phenyl)-isoxazole-3-carboxylic acid ethylamide for either 72 or 96 hours and cell growth was determined by commercially available kits based on either SRB, Alamar blue, methylene blue or WST-1 methods.

### Table 1

| Tumor type | Cell line | $IC_{50}$ (nM) |
| --- | --- | --- |
| Breast | BT474 | 2.8 |
|  | MDA-MB-361 | 6 |
|  | MDA-MB-453 | 3.9 |
|  | SKBr3 | 2.3 |
|  | T47D | 2.6 |
|  | MCF7 | 2.3 |
|  | MDA-MB-231 | 7.7 |
|  | MDA-MB-468 | 3.5 |
| Melanoma | SK-MEL-5 | 3 |
|  | A375 | 3 |
|  | MALME-3M | 7.7 |
|  | SK-MEL-28 | 8 |
|  | WM266.4 | 6.2 |
| Multiple myeloma (MM) | RPMI8226 U266 | 36.7 23.3 |
| Colon | BE | 2.8 |
|  | Colo205 | 6.2 |
|  | HCT116 | 16 |
|  | HT29 | 30 |
|  | MAWI | 50 |
|  | RKO | 3.1 |
| Glioblastoma | U87MG | 6 |
| Head & Neck | HN5 | 8 |
| Leukemia | RPMI-8226 | 6.3 |
| Lung | A549 | 11.7 |
|  | MV522 | 8.1 |
|  | NCI-H1299 | 5.7 |
|  | NCI-H460 | 14 |
| Ovarian | 41M | 3 |
|  | A2780 | 6.1 |
|  | CH1 | 2.8 |
|  | SKOV3 | 3.7 |
| Prostate | PC3 | 5 |
| Stomach | NCI-N87 | 0.2 |
| Gastrointestinal stromal tumour (GIST) | MO7e | 10.6 |
|  | GIST882 | 6.2 |
|  | Baf3 | 22.4 |

**Example 2:** *In vitro* **effects of 5-(2,4-Dihydroxy-5-isopropyl-phenyl)-4-(4-morpholin-4-ylmethyl-phenyl)-isoxazole-3-carboxylic acid ethylamide (AUY922) on a panel of primary human tumor cells.**

The anticancer activity of 5-(2,4-Dihydroxy-5-isopropyl-phenyl)-4-(4-morpholin-4-ylmethyl-phenyl)-isoxazole-3-carboxylic acid ethylamide is evaluated in 30 human tumor xenografts *in vitro* using a clonogenic assay. In this assay, human cells derived from cancer patients are evaluated for the capacity of 5-(2,4-Dihydroxy-5-isopropyl-phenyl)-4-(4-morpholin-4-ylmethyl-phenyl)-isoxazole-3-carboxylic acid ethylamide to inhibit the formation of 3 dimensional colonies. These consist of tumor cells that possess the potential for anchorage independent growth in semisolid medium. The tumor xenografts which have never been cultured in cell culture plastic dishes are isolated from nude mice. Tumor cell suspensions are prepared and incubated in 24 well plates containing layers of soft agar. Under these conditions a special subpopulation of cells selectively grows to colonies. 5-(2,4-Dihydroxy-5-isopropyl-phenyl)-4-(4-morpholin-4-ylmethyl-phenyl)-isoxazole-3-carboxylic acid ethylamide was tested in 6 concentrations up to 10 $\mu$M. The tumor test panel comprises 1 to 6 models of 10 different human tumor or cancer types, which were bladder cancer, colon, liver, non small cell lung (adeno, squamous epithelium and large cell), small cell lung, mammary, ovary, pancreatic, melanoma and pleuramesothelioma. Antitumor effects are recorded as inhibition of colony formation in relation to untreated controls. The concentration which results in 50% reduction in colony formation ($IC_{50}$) are shown in Table 2. Further information on the method has been published (Burger et al., 2004; Fiebig et al., 2004; Smith et al., 2005).

Table 2 shows the concentration (nM) of 5-(2,4-Dihydroxy-5-isopropyl-phenyl)-4-(4-morpholin-4-ylmethyl-phenyl)-isoxazole-3-carboxylic acid ethylamide which inhibits colony formation by 50% ($IC_{50}$). The cells are continually exposed to 5-(2,4-Dihydroxy-5-isopropylphenyl)-4-(4-morpholin-4-ylmethyl-phenyl)-isoxazole-3-carboxylic acid ethylamide and colony formation is determined.

**Table 2**

| Tumor Type | Tumor model | Histology | IC50 (nM) |
|---|---|---|---|
| Bladder | BXF 1218 | Transitional cell carcinoma | 27 |
| | BXF 1228 | Transitional cell carcinoma | 630 |
| Colon | CXF 1103 | Adeno carcinoma | 13 |
| | CXF 158 | Adeno carcinoma | 369 |
| | CXF 1729 | Carcinoma | 467 |
| | CXF 1784 | Carcinoma | 418 |
| | CXF 609 | Adeno carcinoma | 55 |
| Liver | LIXF 575 | Hepatocellular carcinoma | 34 |
| Lung, non- small cell | LXFA 297 | Adeno carcinoma | 28 |
| | LXFA 526 | Adeno carcinoma | 5 |
| | LXFA 629 | Adeno carcinoma | 35 |
| | LXFA 983 | Adeno carcinoma | 126 |
| | LXFE 1422 | Squamous cell carcinoma | 48 |
| | LXFL 1647 | Large cell lung carcinoma | 34 |
| Lung, small cell | LXFS 615 | Small cell lung carcinoma | 30 |
| | LXFS 650 | Small cell lung carcinoma | 2 |
| Breast | MAXF 1162 | Invasive ductal carcinoma | 304 |
| | MAXF 1322 | Pap. adeno carcinoma | 29 |
| | MAXF 1384 | Adeno carcinoma | 209 |
| | MAXF 401 | Pap. adeno carcinoma | 78 |
| | MAXF 583 | Ductual adeno carcinoma | 333 |
| Melanoma | MEXF 1539 | Melanoma | 3 |
| | MEXF 462 | Amelanotic melanoma | 24 |
| | MEXF 535 | Amelanotic melanoma | 43 |
| | MEXF 672 | Amelanotic melanoma | 18 |
| | MEXF 989 | Amelanotic melanoma | 2 |
| Ovary | OVXF 1353 | Adeno carcinoma | 26 |
| | OVXF 1544 | Carcinoma | 53 |
| Pancreas | PAXF 1657 | Adeno carcinoma | 39 |
| Pleuramesothelioma | PXF 1118 | Biphasic pleuramesothelioma | 223 |

**Example 3: Antitumor effect of 5-(2,4-Dihydroxy-5-isopropyl-phenyl)-4-(4-morpholin-4-ylmethyl-phenyl)-isoxazole-3-carboxylic acid ethylamide (AUY922) in the human breast cancer model BT-474**

The estrogen receptor positive cell line BT-474 was initially isolated from a human breast ductal carcinoma established from a solid, invasive ductal carcinoma of the breast obtained from a 60-year-old woman (ATCC number HTB-20). The cells are grown in DMEM high glucose (4.5 g/l) supplemented with 10% FCS, 200 mM L-glutamine and 1% sodium pyruvate.

In preparation for cell inoculation, each mouse is subcutaneously implanted on the upper dorsal side with a 17β-Estradiol pellet (25 μg/day; 90 day release) using a trocar needle. BT-474 cells (5x10^6) are injected in 200 μl Matrigel:HBSS (1:1 vol) (BD Matrigel™ Basement Membrane Matrix). The injection site is subcutaneously in the right flank. Treatment with AUY922 is initiated when the average tumor volume reached approximately 100 mm$^3$. Tumor growth is monitored at regular intervals. The xenograft tumor sizes are measured manually with calipers and the tumor volume is estimated using the formula: (W x L x H x $\pi/6$), where width (W), height (H) and length (L) are the three largest diameters.

Results are presented as mean ± SEM. Tumor data are analyzed by ANOVA with post hoc Dunnet's test for comparison of treatment versus control group. As a measure of efficacy the %T/C value is calculated at the end of the experiment according to:

$$(\Delta \text{tumor volume}_{treated}/\Delta \text{tumor volume}_{control})*100$$

where Δtumor volumes represent the mean tumor volume on the evaluation day minus the mean tumor volume at the start of the experiment.

The antitumor effect of AUY922 is evaluated in the BT-474 xenograft model. In this study, the treatment period is 21 days. Each group consists of eight tumor bearing animals. At the end of the study, the tumor sizes in the treatment groups are compared to those of the vehicle treated groups and the effect is expressed as %T/C. Statistically significant reduction of tumor sizes are observed when AUY922 is administered once per week at 17-25 mg/kg (Table 3).

**Table 3: Effect of AUY922 on BT-474 xenograft growth**

| Compound | Dose, schedule, route | T/C (%) | ΔTumor volume (mm$^3$) |
|---|---|---|---|
| Vehicle control | 10 ml/kg, qw, i.v. | 100 | 528 ± 123 |
| AUY922 | 8.3 mg/kg, qw, i.v. | 43 | 229 ± 73 |
| AUY922 | 17 mg/kg, qw, i.v. | 9 | 46 ± 27* |
| AUY922 | 25 mg/kg, qw, i.v. | 3 | 15 ± 23* |
| * P < 0.05; one-way ANOVA *post hoc* Dunnet's test. | | | |

**Example 4: Antitumor effect of 5-(2,4-Dihydroxy-5-isopropyl-phenyl)-4-(4-morpholin-4-ylmethyl-phenyl)-isoxazole-3-carboxylic acid ethylamide (AUY922) in the rat breast cancer model BN-472**

The transplantable rat breast cancer tumor BN472 is serially passaged as fragments in female syngeneic Brown Norway rats. The injection site is orthotopically in the mammary fat pad. Treatment with AUY922 is initiated when the average tumor volume reaches approximately 100 mm$^3$. Tumor growth is monitored at regular intervals. The xenograft tumor sizes are measured manually with calipers and the tumor volume is estimated using the formula: (W x L$^2$ x $\pi/6$), where width (W) and height (H) are the two largest diameters. Results are presented as mean ± SEM. Tumor data were analyzed by ANOVA with *post hoc* Dunnet's test for comparison of treatment versus control group. As a measure of efficacy the %T/C value is calculated at the end of the experiment according to:

$$(\Delta \text{tumor volume}_{treated}/\Delta \text{tumor volume}_{control})*100$$

where Δtumor volumes represent the mean tumor volume on the evaluation day minus the mean tumor volume at the start of the experiment.

The antitumor effect of AUY922 is evaluated in the BN472 xenograft model. Each group consists of seven tumor bearing animals. At the end of the study, the tumor sizes in the treatment groups are compared to those of the vehicle treated groups and the effect is expressed as %T/C. Statistically significant reduction of tumor sizes is observed when AUY922 was administered once per week at 50 mg/kg (Table 4).

**Table 4: Effect of AUY922 on BN472 xenograft growth**

| Compound | Dose, schedule, route | T/C (%) | $\Delta$Tumor volume ($mm^3$) |
|---|---|---|---|
| Vehicle control | 2 ml/kg, qw, i.v. | 100 | 5569 $\pm$ 1639 |
| AUY922 | 25 mg/kg, qw, i.v. | 78 | 4357 $\pm$ 1338 |
| AUY922 | 50 mg/kg, qw, i.v. | 21 | 1148 $\pm$ 152* |
| * P < 0.05; one-way ANOVA *post hoc* Dunnet's test. | | | |

**Example 5: Antitumor effect of 5-(2,4-Dihydroxy-5-isopropyl-phenyl)-4-(4-morpholin-4-ylmethyl-phenyl)-isoxazole-3-carboxylic acid ethylamide (AUY922) in the rat pancreatic cancer model CA20948**

The transplantable rat pancreatic tumor CA20948 is serially passaged as cell homogenates in male syngeneic Lewis rats. The injection site is subcutaneously on the right flank. Treatment with AUY922 is initiated when the average tumor volume reaches approximately 100 $mm^3$. Tumor growth is monitored at regular intervals. The xenograft tumor sizes is measured manually with calipers and the tumor volume is estimated using the formula: (W x $L^2$ x $\pi$/6), where width (W) and height (H) are the two largest diameters.

Results are presented as mean $\pm$ SEM. Tumor data were analyzed by ANOVA with *post hoc* Dunnet's test for comparison of treatment versus control group. As a measure of efficacy the %T/C value is calculated at the end of the experiment according to:

$$(\Delta \text{tumor volume}_{treated}/\Delta \text{tumor volume}_{control})*100$$

where $\Delta$tumor volumes represent the mean tumor volume on the evaluation day minus the mean tumor volume at the start of the experiment.

The antitumor effect of AUY922 is evaluated in the CA20948 xenograft model. Each group consisted of six tumor bearing animals. At the end of the study, the tumor sizes in the treatment groups are compared to those of the vehicle treated groups and the effect is expressed as %T/C. Statistically significant reduction of tumor sizes is observed when AUY922 is administered once per week at 50 and 75 mg/kg (Table 5).

**Table 5: Effect of AUY922 on CA20948 xenograft growth**

| Compound | Dose, schedule, route | T/C (%) | $\Delta$Tumor volume ($mm^3$) |
|---|---|---|---|
| Vehicle control | 2 ml/kg, qw, i.v. | 100 | 23267 $\pm$ 7810 |
| AUY922 | 50 mg/kg, qw, i.v. | 30 | 7090 $\pm$ 2553* |
| AUY922 | 75 mg/kg, qw, i.v | 21 | 4796 $\pm$ 1354* |
| * P < 0.05; one-way ANOVA *post hoc* Dunnet's test. | | | |

**Claims**

1. The use of 5-(2,4-Dihydroxy-5-isopropyl-phenyl)-4-(4-morpholin-4-ylmethyl-phenyl)isoxazole-3-carboxylic acid ethylamide or a pharmaceutically acceptable salt, hydrate or solvate thereof, for the manufacture of a pharmaceutical composition for the treatment of cancer of the lung.

2. The use of 5-(2,4-Dihydroxy-5-isopropyl-phenyl)-4-(4-morpholin-4-ylmethyl-phenyl)isoxazole-3-carboxylic acid ethylamide, or a pharmaceutically acceptable salt thereof, according to claim 1.

3. 5-(2,4-Dihydroxy-5-isopropyl-phenyl)-4-(4-morpholin-4-ylmethyl-phenyl)-isoxazole-3-carboxylic acid ethylamide or a pharmaceutically acceptable salt, hydrate or solvate thereof, for use in treating cancer of the lung.

4. 5-(2,4-Dihydroxy-5-isopropyl-phenyl)-4-(4-morpholin-4-ylmethyl-phenyl)-isoxazole-3-carboxylic acid ethylamide, or a pharmaceutically acceptable salt thereof, for use according to claim 3.

5. A pharmaceutical preparation comprising 5-(2,4-Dihydroxy-5-isopropyl-phenyl)-4-(4-morpholin-4-ylmethyl-phenyl)-isoxazole-3-carboxylic acid ethylamide or a pharmaceutically acceptable salt, hydrate or solvate thereof and at least one pharmaceutically acceptable carrier, for use in the treatment of cancer of the lung.

Europäisches
Patentamt

European
Patent Office

Office européen
des brevets

# EUROPEAN SEARCH REPORT

Application Number

EP 18 18 7344

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DATABASE ADISCTI [Online] ADIS; 13 September 2007 (2007-09-13), US NATIONAL INSTITUTES OF HEALTH: "AUY922 : therapeutic use Advanced breast cancer Phase I/II trial in patients with either HER2-positive or oestrogen receptor-positive locally advanced or metastatic disease", XP002466385, retrieved from STN Database accession no. 2007:8882 * abstract * | 1-5 | INV.<br>A61P35/00<br>A61K31/5377 |
| A | US 2007/105862 A1 (BRUNCKO MILAN [US] ET AL) 10 May 2007 (2007-05-10) * page 1; claims * | 1-5 | |
| A | WO 2006/113498 A (CHIRON CORP [US]; MACHAJEWSKI TIMOTHY D [US]; GAO ZHENHAI [US]; LEVINE) 26 October 2006 (2006-10-26) * paragraphs [0329], [0330]; claims 34-38 * | 1-5 | |
| A,D | WO 2004/072051 A (VERNALIS CAMBRIDGE LTD [GB]; CANCER REC TECH LTD [GB]; INST OF CANCER) 26 August 2004 (2004-08-26) * claims 1,31; example 78 * | 1-5 | TECHNICAL FIELDS SEARCHED (IPC)<br><br>A61K<br>A61P |

The present search report has been drawn up for all claims

| Place of search | Date of completion of the search | Examiner |
|---|---|---|
| Munich | 10 October 2018 | Blott, Catherine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**

EP 18 18 7344

This annex lists the patent family members relating to the patent documents cited in the above-mentioned European search report.
The members are as contained in the European Patent Office EDP file on
The European Patent Office is in no way liable for these particulars which are merely given for the purpose of information.

10-10-2018

| Patent document cited in search report | | Publication date | Patent family member(s) | Publication date |
|---|---|---|---|---|
| US 2007105862 A1 | | 10-05-2007 | NONE | |
| WO 2006113498 A | | 26-10-2006 | --------- | |
| WO 2004072051 A | | 26-08-2004 | --------- | |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004072051 A